# EUROPEAN PATENT SPECIFICATION

(11) **EP 0 348 144 B1**
(45) Date of publication and mention of the grant of the patent: **10.01.1996**
(21) Application number: 89306211.7
(22) Date of filing: 20.06.1989
(51) Int. Cl.: G01N 33/53, G01N 33/571

(54) **Method for treating mucus-containing immunoassay samples**
Verfahren zur Behandlung von Immunotest-Proben, die Schleim enthalten
Procédé pour traiter des échantillons d'essai immunologique contenant du mucus

(30) Priority: 22.06.1988 US 210108
(43) Date of publication of application: 27.12.1989
(73) Proprietor: Hygeia Sciences, Inc., Newton Massachusetts 02160-1432 (US)
(72) Inventor: Berke, Carl M., Boston, MA 02116 (US); Penoyer, R. Scott, Newburyport, MA 01950 (US); Wong, Tailap Philip, Westwood, MA 02090 (US)
(74) Representative: Moon, Donald Keith

(56) References cited:
- EP-A- 0 103 958
- EP-A- 0 141 244
- EP-A- 0 250 991
- WO-A-86/05592
- US-A- 4 497 900
- US-A- 4 703 001

## Description

This invention relates to immunoassay for antigens in samples which include mucus.

Immunoassays can be used in medical diagnosis for determination of the presence of a specific bacterium. One form of immunoassay involves detection of a specific bacterial antigen using a labeled antibody. For example, the antibody may be labeled with detectable enzyme, such as horseradish peroxidase, so that the presence of the antibodies can be detected by providing substrate for the enzyme, and detecting conversion of the substrate to a product.

One problem encountered in immunoassay procedures involves inaccuracies caused by interference from other substances in the sample. For example, false positives can arise from non-specific binding between mucus in the sample and the labeled antibody. Mucus may be naturally present, for example, in urogenital specimens collected for the purpose of diagnosing venereal disease by immunoassay. In addition, mucus can physically interfere with the ability of immunoassay to detect specimen constituents, when the analyte of interest is encrypted in the sample matrix thereby is less accessible to the test kit reagents. A well-known method for the preparation of respiratory sputum samples for diagnostic testing involves the use of mucolytic agents, such as N-acetylcysteine or dithiothreitol, to reduce the viscosity of, and to better homogenize, the specimen. These agents which are the principle constituent of the mucus, by dissociating disulfide bonds. These agents, based on thiol-mediated dissociation, are generally unsuitable for immunoassays that use heme-containing enzyme labels, such as horseradish peroxides, because of their inhibitory effect on the enzyme.

An immunoassay for the detection of Neisseria gonorrhoeae in clinical samples is described in US patent No. 4497900.

EP-A-103958 describes the use of periodate impregnated onto a solid support to reduce interference by ascorbate in peroxidase-dye assay systems.

We have found that it is possible to reduce interference from mucus in the sample, without unacceptable detrimental effects on the immunoassay, by subjecting the sample to oxidative conditions. Thus, the invention features a method and kit for immunoassay using an antibody to bind an antigen in a sample having a mucus matrix. The method includes the step of treating the mucus-containing sample with an oxidant at a concentration sufficient to prevent non-specific binding of the antibody to the mucus matrix. After this treatment the sample is processed according to standard assay techniques.

In preferred embodiments, oxidation is accomplished by treatment with hydrogen peroxide or an organic peroxide; the sample is a urogenital (particularly an endocervical) specimen; and the antigen to be detected is a Neisseria gonorrhoeae, antigen for example the Neisseria gonorrheae antigen H-8. The kit preferably includes means to collect an endocervical sample (e.g. a swab).

This invention provides a method by which the number of the false positives in an immunoassay is reduced, without unacceptable detrimental effects on binding of the antigen and antibody in the assay, or on the ability to detect the presence of low levels of antigen.

The choice of peroxide as oxidant is particularly advantageous because of its tendency to decompose in the presence of catalase or pseudoperoxidase activity that is naturally present in urogenital samples. Such enzymatic activity arises from the presence of normal commensal microflora and constituents of blood or epithelial cells that are unavoidably present in such samples. This sample-activated decomposition of hydrogen peroxide is advantageous because oxidants, such as hydrogen peroxide, can damage the enzyme and thereby reduce label activity. However, the hydrogen peroxide level is reduced by the above-described activity during sample incubation, so relatively high levels of peroxide may be employed without the need for an additional neutralizing step before the addition of enzyme label. Stronger oxidants than peroxide, such as periodate or perchlorate, are less suitable for enzyme immunoassay, because of their deleterious effect on the enzyme label in the absence of any neutralizing agent.

This invention is particularly suitable for detection of characteristic antigens derived from Neisseria gonorrhoeae, Chlamydia trachomatis, and herpes virus.

Other features and advantages of the invention will be apparent from the following description of the preferred embodiment thereof, and from the claims.

This invention concerns the pretreatment of a sample for enzyme immunoassay. Generally this involves mixing the specimen with a minimum volume of an oxidative mucolytic reagent, such as hydrogen peroxide, at a concentration ranging from 1-15% (w/w) for 1-5 minutes before performing a standard immunoassay.

Sexually transmitted disease (STD) specimens from female patients are generally problematic in enzyme labeled immunoassay techniques because of the high level of false positives. However, they are particularly suited to the assay procedure of this invention.

### EXAMPLE: Assay for Gonococcal Antigen

All of the following operations are performed at ambient temperature (about 20°C).
1. A urogenital swab specimen was placed in a glass tube for 5 minutes with 100 ul of a 5% solution of hydrogen peroxide in citrate phosphate buffer at pH 5 (49 mM citric acid and 0.1 M dibasic sodium phosphate).
2. The swab specimen was then placed in 500 ul of assay buffer, composed of 0.45 M potassium phosphate, 0.113 M sodium chloride, 0.56% bovine serum albumin, 0.338% Triton X-100 and 0.056% thimerosol at pH 7.2. The swab was agitated in the solution at the end of the extraction. By this process, gonococcal antigen is extracted from the organism and specimen matrix into the solution.
3. After expressing liquid from the swab, the swab was discarded.
4. To the tube containing expressed sample fluid, 50 ul of antigonococcus antibody covalently linked to horseradish peroxidase (by the method of Wilson and Nakane, in Immunofluorescence and Related Staining Techniques, Knapp et al., eds., 1978. pp. 215-224) is added. The tube was agitated to mix the reagents thoroughly.
5. A dipstick coated with anti-gonococcus antibody, (by the method of Catt and Treager, Science 158:1570 (1967)), was then added to the tube for 30 minutes.
6. The dipstick was washed thoroughly under a stream of tap water for 5 seconds, then shaken to remove excess water.
7. The washed dipstick was then placed in a new glass tube containing 237 ul of a fresh chromogenic developer, composed of 3 parts 5.2 mM 3,3', 5,5' - tetramethylbenzidine in methanol to 4 parts of 0.03% hydrogen peroxide in 49 mM citric acid, 0.1 M dibasic sodium phosphate, 0.006% sodium stannate and 0.005% thimerosol at pH 5. See, Gerber et al. U.S. Pat. 4,503.143.
8. After a 10 minute incubation, the dipstick was removed from the developer. The intensity of blue color in the developer is proportional to the quantity of gonococcal antigen present in the sample. The absence of blue indicates the absence of gonococcal antigen.

Results from the above assay were compared with that from standard culture techniques. Addition of the hydrogen peroxide treatment step increased both the sensitivity and specificity of the assay. The sensitivity is defined as the number of positives detected by the immunoassay techniques versus the number of positives detected by culture techniques; and specificity is defined as the ratio of number of negatives detected by the immunoassay techniques compared to the number detected by the culture techniques.

In particular, matched pairs of specimens were collected from individual patients. In each case individual swabs were characterized by first streaking a standard growth medium to obtain a reference result for gonococcal culture. One member of the pair was subjected to the above-described peroxide treatment, and the other was processed by standard immunoassay protocol.

The peroxide treatment improves agreement between enzyme immunoassay and culture for both positive and negative samples. Clinical sensitivity was not deleteriously affected, and specificity was enhanced.

In one experiment, conducted generally as described above, agreement between enzyme immunoassay (EIA) and culture was improved for both positive samples (sensitivity) and negative samples (specificity).

| | Sensitivity | Specificity | |
|---|---|---|---|
| | EIA+/culture+ | EIA-/culture- | |
| +perox | 9/11 = 82% | 51/55 | 93% |
| -perox | 6/11 = 55% | 48/55 | 87% |

Another benefit of peroxide treatment is reduction of background enzyme activity, and a resulting improvement in clinical specificity. In particular, the background level of enzyme activity in the immunoassay was measured for matched pairs of culture-negative samples. Each member of the pair was treated with extended incubation times to accentuate signal levels, either with peroxide or without peroxide and then read visually and quantitated by spectrophotometry. Peroxide treatment substantially reduced the background signal in culture-negative samples. Results are tabulated below:

| | -perox | +perox |
|---|---|---|
| EIA-/cult- | 24/38 (63%) | 36/38 (96%) |
| Mean OD | 0.10 | 0.02 |

The target antigen is substantially unaffected by the oxidative conditions produced by the peroxide treatment. When standard laboratory preparations of gonococcal organism suspensions were assayed in the immunoassay, the effect of hydrogen peroxide pretreatment (100 microliters at 5% for 5 minutes) did not significantly affect signal intensity.

Other embodiments are within the following claims. For example other peroxides are also useful in this invention, such as organic peroxides, including methyl hydroperoxide, t-butyl hydroperoxide, cumene hydroperoxide.

## Claims

1. A method of immunoassay using an antibody to bind to an antigen in a sample comprising a mucus matrix, said method comprising the steps of:
first treating said mucus containing sample with an oxidant, present at a concentration sufficient to reduce non-specific binding of said antibody to said mucus matrix and then
contacting said antibody with said sample and detecting antibody-antigen binding in said sample.

2. A method according to claim 1 wherein sample mucus is oxidised with hydrogen peroxide or an organic peroxide.

3. A method according to any one of the preceding claims wherein said sample is a urogenital sample.

4. A method according to any one of the preceding claims wherein said antigen is a Neisseria gonorrhoeae antigen.

5. A method according to claim 4 wherein the antigen is H-8 antigen.

6. A method according to any one of claims 1 to 5 in which the sample comprises microflora or other constituents exhibiting pseudoperoxidase activity.

7. A kit for an immunoassay using an antibody to bind an antigen in a sample comprising a mucus matrix, said kit comprising:
an oxidant, and
an antibody labelled with a detectable label.

8. A kit according to claim 7 wherein said oxidant is hydrogen peroxide or an organic peroxide.

9. A kit according to claim 7 or claim 8 wherein said kit comprises means to collect a urogenital sample.

10. A kit according to any one of claims 7 to 9 wherein said antibody is one that specifically binds to a Neisseria gonorrhoeae antigen.

11. A kit according to claim 10 wherein said antigen is H-8 antigen.

## Patentansprüche

1. Immunotestverfahren unter Verwendung eines Antikörpers, der an ein Antigen in einer Probe bindet, die eine Schleimmatrix enthält, wobei es zu dem Verfahren gehört:
daß zunächst die den Schleim enthaltende Probe mit einem Oxidationsmittel behandelt wird, das in einer Konzentration vorliegt, die ausreicht, die unspezifische Bindung des Antikörpers an die Schleimmatrix zu reduzieren und
daß sodann der Antikörper mit der Probe in Berührung gebracht wird und die Antikörper-Antigenbindung in der Probe erkannt wird.

2. Verfahren nach Anspruch 1, bei dem die Schleimprobe mit Wasserstoffperoxid oder einem organischen Peroxid oxidiert wird.

3. Verfahren nach einem der vorhergehenden Ansprüche, bei dem die Probe aus dem Urogenitalbereich stammt.

4. Verfahren nach einem der vorhergehenden Ansprüche, bei dem das Antigen ein Neisseria gonorrhoea-Antigen ist.

5. Verfahren nach Anspruch 4, bei dem das Antigen ein H-8-Antigen ist.

6. Verfahren nach einem der Ansprüche 1 bis 5, bei dem die Probe eine Mikroflora oder andere Bestandteile aufweist, die eine Pseudoperoxidaseaktivität zeigt bzw. zeigen.

7. Zusammenstellung für einen Immunotest unter Verwendung eines Antikörpers, der an ein Antigen in einer Probe bindet, die eine Schleimmatrix enthält, wobei die Zusammenstellung enthält:
ein Oxidationsmittel und
einen mit einer erkennbaren Markierung markierten Antikörper.

8. Zusammenstellung nach Anspruch 7, bei der das Oxidationsmittel Wasserstoffperoxid oder ein organisches Peroxid ist.

9. Zusammenstellung nach Anspruch 7 oder 8, bei dem die Zusammenstellung Mittel enthält, um eine Probe aus dem Urogenitalbereich zu nehmen.

10. Zusammenstellung nach einem der Ansprüche 7 bis 9, bei dem der Antikörper ein solcher ist, der speziell an ein Neisseria gonorrhoea-Antigen bindet.

11. Zusammenstellung nach Anspruch 10, bei der das Antigen ein H-8-Antigen ist.

## Revendications

1. Procédé d'immunodétection mettant en oeuvre un anticorps se fixant à un antigène dans un échantillon qui contient une matrice de mucus, ledit procédé comprenant les étapes de :
traitement tout d'abord dudit échantillon qui contient le mucus avec un oxydant, présent en une teneur suffisante pour réduire la fixation non spécifique dudit anticorps à ladite matrice de mucus puis de
mise en contact dudit anticorps avec ledit échantillon et de détection de la combinaison anticorps-antigène dans ledit échantillon.

2. Procédé selon la revendication 1, où le mucus de l'échantillon est oxydé avec de l'eau oxygénée ou un peroxyde organique.

3. Procédé selon l'une quelconque des revendications précédentes, où ledit échantillon est un échantillon urogénital.

4. Procédé selon l'une quelconque des revendications précédentes, où ledit antigène est un antigène de Neisseria gonorrhoeae.

5. Procédé selon la revendication 4, où l'antigène est l'antigène H-8.

6. Procédé selon l'une quelconque des revendications 1 à 5, dans lequel l'échantillon renferme une microflore ou d'autres composants présentant une activité pseudoperoxydasique.

7. Coffret d'immunodiagnostic pour la mise en oeuvre d'un anticorps se combinant à un antigène dans un échantillon comprenant une matrice de mucus, ledit coffret contenant :
un oxydant et
un anticorps marqué à l'aide d'un marqueur détectable.

8. Coffret selon la revendication 7, où ledit oxydant est l'eau oxygénée ou un peroxyde organique.

9. Coffret selon la revendication 7 ou le revendication 8, où ledit coffret comprend un moyen pour prélever un échantillon urogénital.

10. Coffret selon l'une quelconque des revendications 7 à 9, où ledit anticorps est tel qu'il se combine à un antigène de Neisseria gonorrhoeae.

11. Coffret selon la revendication 10, où ledit antigène est l'antigène H-8.
